# EUROPEAN PATENT SPECIFICATION

(11) **EP 3 991 685 B1**
(45) Date of publication and mention of the grant of the patent: **26.08.2026**
(21) Application number: 20205398.9
(22) Date of filing: 03.11.2020
(51) Int. Cl.: A61B 90/20, A61B 90/25, A61B 90/00

(54) **SURGICAL MICROSCOPE SYSTEM**
CHIRURGISCHES MIKROSKOPSYSTEM
SYSTÈME DE MICROSCOPE CHIRURGICAL

(43) Date of publication of application: 04.05.2022
(73) Proprietor: Leica Instruments (Singapore) Pte. Ltd., 608924 Singapore (SG)
(72) Inventor: KOK, Alvin, 085301 Singapore (SG); WANG, Ken, 608924 Singapore (SG); SCHUTZ, Marco, 338986 Singapore (SG); CHUA, Li Nah, 608924 Singapore (SG); PORTILLA, Oscar, 608924 Singapore (SG)
(74) Representative: 2SPL Patentanwälte PartG mbB

(56) References cited:
- EP-A1- 1 972 294
- EP-A1- 3 295 890
- EP-A1- 3 527 123
- WO-A1-2019/167528
- US-A1- 2015 301 326
- ANONYMOUS: "AUGMENT YOUR REALITY Introducing the ARveo digital augmented reality microscope", 3 October 2019 (2019-10-03), XP055791340, Retrieved from the Internet <URL:https://downloads.leica-microsystems.com/ARveo/Brochures/ARveo_Brochure_MC-0000311_EN.pdf> [retrieved on 20210330]
- ANONYMOUS: "Premium Surgical Microscope STAY FOCUSED FOR BEST RESULTS Leica M530 OH6 with FusionOptics", 14 October 2019 (2019-10-14), pages 1 - 16, XP055791663, Retrieved from the Internet <URL:https://downloads.leica-microsystems.com/Leica%20M530%20OH6/Brochures/Leica_M530_OH6_Brochure_MC-0000315_EN.pdf> [retrieved on 20210330]

## Description

### Field

Various aspects of the present disclosure relate to a surgical microscope system, and in particular, to an arm for a surgical microscope system.

### Background

As surgery moves towards a more digital workflow with the introduction of advanced surgical guidance technologies, one of the trends moves towards a "surgical cockpit" concept, whereby the surgeon operates with precise big-screen 3D visualization of tissue structures, integrated with on-screen surgical data captured with a single line of sight (as opposed to looking in the binoculars and then at the screen for data, which may break concentration).

One of the main value propositions of the 3D heads-up approach to surgical microscopy relates to improvements of the surgeon's ergonomics due to the microscope stack height. 3D heads-up viewing allows the surgeon to comfortably visualize the surgical workflow with better ergonomics and natural posture (increased surgeon comfort) while observing on-screen real-time surgical data to support an interconnected workflow (increased surgical confidence). With the integration of optical accessories into the optics carrier (inverters, image injection, optical coherence tomography, laser filters, camera bridge, retinal viewing accessories etc.), the large stack height is often a common pain point faced by surgeons using the traditional oculars. Accordingly, this may result in an uncomfortable, non-ergonomic position due to the large microscope stack height. Moreover, the arm of the optics carrier presents an obstacle in allowing the surgeons to look forward. The 3D heads-up monitor is often positioned at an angle off-center to the right due to obstruction of surgeon's left field of view by the microscope optics carrier arm. This results in the surgeon's head tilting at an angle during surgery, which may worsen the ergonomics of the system.

EP 3 527 123 A1 relates to a medical observation device, such as a microscope or endoscope with an arm and a display device, and an image processing method.

A 2019 brochure of the ARveo digital augmented reality microscope (available at https://downloads.leica-microsystems.com/ARveo/Brochures/ARveo Brochure MC-0000311_EN.pdf) by Leica Microsystems shows a digital AR microscope with an arm and a display device.

WO 2019/167528 A1 relates to a medical observation instrument equipped with an arm which is formed by coupling a plurality of links to each other via joints, and which has at least three degrees of freedom enabled by rotational movements about rotational axes, an imaging device supported by the arm, and an arm control unit which controls movement of the arm. Images generated by the imaging device are shown via a display.

### Summary

There may be a desire to provide an improved concept for a microscope system, which improves ergonomics for the surgeon.

The present invention is defined by appended claims 1 and 13. Specific embodiments are set forth in the dependent claims.

Various embodiments of the present disclosure are based on the finding, that the ergonomics of the use of a surgical microscope system can be improved, for a surgeon, by providing the surgeon with a true frontal field of view, for an unobstructed visualisation of the surgical area. Embodiments may thus provide true frontal viewing of surgical cockpits. In general, surgical microscope system comprise an arm that is attached both to the optics carrier (i.e. the actual microscope) and a base unit or ceiling mount. This arm may comprise a first section (also denoted "parallelogram section"), which can be used to control the vertical height above ground of the optics carrier, and a second section (or "optics carrier arm", "optics carrier section"), which is arranged between the first section and the optics carrier. In general, the second section may be used to provide a fine-grained adjustment of the orientation of the optics carrier, and/or a rotational adjustment. In various embodiments of the present disclosure, this second section is adapted in manner that creates a free space, freeing up the field of view of the surgeon towards a display device of the surgical microscope system. In general, this may be achieved by redesigning the second section into an extended "C-shaped or V-shaped" structure to allow a wider field of view over the microscope stack.

According to the invention, a surgical microscope system comprises an optics carrier, the optics carrier comprising optical components of a microscope of the surgical microscope system. The surgical microscope system comprises a display device for displaying image data recorded by an optical imaging sensor of the surgical microscope system. The surgical microscope system comprises an arm. The arm comprises a first section and a second section. The first section is configured to provide a vertical and/or lateral adjustment of the position of the optics carrier. The second section is attached to a suspension point of the first section and to the optics carrier. The second section has a shape that creates a free space around a geometric axis between the suspension point and the optics carrier, such that an optical path between a central area of the display device and a surgeon being located at the opposite side of the optics carrier remains unobstructed by the second section. The optical path intersects the geometric axis between the suspension point and the optics carrier. By using a second section with a shape that is adapted in this manner, the field of view of the surgeon may be left mostly unobstructed, such that the surgeon has a good view on the display device without straining their neck.

In general, the central area may be centered around a center point of the display device. For example, the central area may occupy at least 20% of an overall area of the display device. In general, the portion of the surgical site that is in focus is displayed within the central area of the display device. The larger the central area that can be seen without obstructions by the surgeon, the more useful the surgical cockpit becomes.

In general, such an unobstructed view may be achieved in various ways. According to the invention, the shape of the second section comprises a first sub-section that extends laterally outwards from the suspension point, a second sub-section that leads downwards from the first sub-section, and a third sub-section that subsequently returns inwards from the second sub-section towards the optics carrier. This may move the second sub-section out of the way, such that the optical path towards the display device allows for a broader field of view. In general, the above-described shape may be likened to a "C"-shape of "lying V"-shape. In other words, the second section may be substantially shaped like the letter "C" or like a lying letter "V".

In various examples, the first sub-section extends outwards for at least 15 cm. This may create the fee space around the optical path. At the other end, the third sub-section may return inwards for a similar distance, e.g. minus the space occupied by the optics carrier. This may determine the width of the central area that is visible in an unobstructed manner by the surgeon.

According to the invention, the second sub-section leads downwards for at least 20 cm. For example, the second sub-section may comprise a portion that leads straight downwards. This portion may occupy at least 40% of a vertical height of the second sub-section. The height of the second sub-section, and in particular the height of the portion that leads straight downwards, may determine the height of the central area of the display device.

For example, a transition between the first and second sub-section may be slanted, such that, in a parked position of the first and second section of the arm of the surgical microscope system, a pre-defined distance remains between the first section and the slanted transition between the first and second sub-section of the second section. Consequently, the second section may remain freely rotatable in a parked position of the arm of the surgical microscope system.

**In** some examples, a vertical height of the third sub-section is at least 20% larger than a lateral width of the third sub-section. This may provide an improved stability of the attachment of the optical carrier to the arm of the surgical microscope system.

**In** general, various types of display may be used - e.g. displays that are attached to the base unit of the surgical microscope system, or displays that are attached to a wall of the operating room. **In** both cases, a three-dimensional viewing experience may be achieved, e.g. by using polarized glasses. Accordingly, the display device may be a display device for displaying three-dimensional image data.

In some examples, the optics carrier is an optics carrier without an optical eyepiece. This may create a field of view that allows for a large portion of the field of view of the surgeon to remain unobstructed. Alternatively, the optics carrier may comprise an optical or hybrid eyepiece for the main surgeon, and, further optionally, a digital eyepiece for an assistant.

In various examples, the shape of the second section is such, that a first lateral distance between a sub-section of the second section that is attached to the optics carrier and the display device is smaller than a lateral distance between a sub-section of the second section that is attached to the first section and the display device. In other words, the bottom portion of the second section may be slanted towards the display device, leaving some space for a digital eyepiece for an assistant. Accordingly, the optics carrier may comprise, at a side of the optics carrier the second section is attached at, a digital eyepiece (e.g. for the assistant).

In general, the optics carrier may comprise optical imaging devices for generating image data that is to be displayed on the display device, and various other components. These components may require an electrical and/or data connection to the base unit of the surgical microscope system. The cables being used for the electrical and/or data connection may lead through the first and second section of the arm. For example, the second section may comprise a cable duct and a removable cover for accessing cables within the cable duct, e.g. for quick access to the cables being lead between the base unit and the optics carrier.

In some embodiments, the second section is adapted to rotate around the suspension point. For example, the second section may be adapted to rotate around the suspension point, such that a major sub-section of the second section (e.g. the second sub-section, as shown in the following) can be positioned on the left side or on the right side of the optical axis, from the perspective of the surgeon. This may allow the assistant surgeon sitting on the side to comfortably view the 3D monitor without obstruction to their view.

Various embodiments of the present disclosure provide a method for a surgical microscope system. The method comprises providing an optics carrier of the surgical microscope system, the optics carrier comprising optical components of a microscope of the surgical microscope system. The method comprises providing a display device of the surgical microscope system, the display device being suitable for displaying image data recorded by an optical imaging sensor of the surgical microscope system. The method comprises providing an arm of the surgical microscope system. The arm comprises a first section and a second section. The first section is configured to provide a vertical and/or lateral adjustment of the position of the optics carrier. The second section is attached to a suspension point of the first section and to the optics carrier. The arm is provided with the second section having a shape that creates a free space around a geometric axis between the suspension point and the optics carrier, such that an optical path between a central area of the display device and a surgeon being located at the opposite side of the optics carrier remains unobstructed by the second section, the optical path intersecting the geometric axis between the suspension point and the optics carrier. The display device being provided at a side of the optics carrier that is opposite a desired position of the surgeon.

### Brief description of the Figures

Some examples of apparatuses and/or methods will be described in the following by way of example only, and with reference to the accompanying figures, in which
- Fig. 1a: shows a schematic diagram of an example of a surgical microscope system;
- Figs. 1b to 1e: show schematic diagrams of examples of a microscope system, where a field of view of a surgeon system is shown;
- Fig. 1f: shows a schematic diagram of an example of a surgical microscope system with an arm comprising a first and a second section, where the second section can be rotated around a suspension point that is part of the first section;
- Figs. 2a to 2c: show schematic diagrams of an example of a surgical microscope system with two eyepieces from different perspectives;
- Figs. 3a to 3h: show different shapes of portions of a second section of an arm of a surgical microscope system;
- Fig. 4: illustrates a field of view of a surgeon according to an example;
- Figs. 5a to 5c: show schematic diagrams of a second section of an arm of a surgical microscope system from different perspectives;
- Fig. 6: shows a schematic diagram of a second section of an arm of a surgical microscope with a removable cover; and
- Fig. 7: shows a flow chart of a method for a surgical microscope system.

### Detailed Description

Some examples are now described in more detail with reference to the enclosed figures. However, other possible examples are not limited to the features of these embodiments described in detail. Other examples may include modifications of the features as well as equivalents and alternatives to the features. Furthermore, the terminology used herein to describe certain examples should not be restrictive of further possible examples.

Throughout the description of the figures same or similar reference numerals refer to same or similar elements and/or features, which may be identical or implemented in a modified form while providing the same or a similar function. The thickness of lines, layers and/or areas in the figures may also be exaggerated for clarification.

When two elements A and B are combined using an 'or', this is to be understood as disclosing all possible combinations, i.e. only A, only B as well as A and B, unless expressly defined otherwise in the individual case. As an alternative wording for the same combinations, "at least one of A and B" or "A and/or B" may be used. This applies equivalently to combinations of more than two elements.

If a singular form, such as "a", "an" and "the" is used and the use of only a single element is not defined as mandatory either explicitly or implicitly, further examples may also use several elements to implement the same function. If a function is described below as implemented using multiple elements, further examples may implement the same function using a single element or a single processing entity. It is further understood that the terms "include", "including", "comprise" and/or "comprising", when used, describe the presence of the specified features, integers, steps, operations, processes, elements, components and/or a group thereof, but do not exclude the presence or addition of one or more other features, integers, steps, operations, processes, elements, components and/or a group thereof.

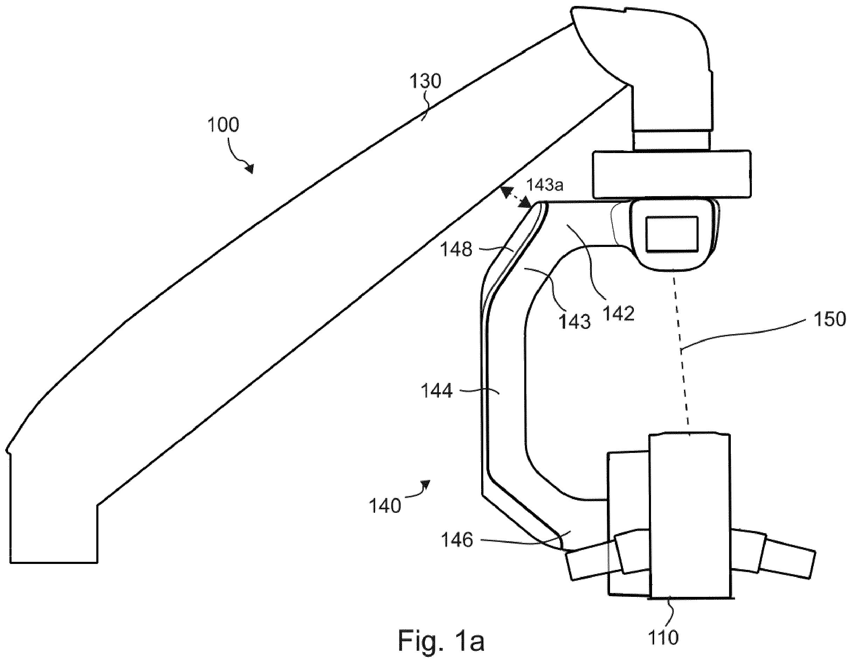

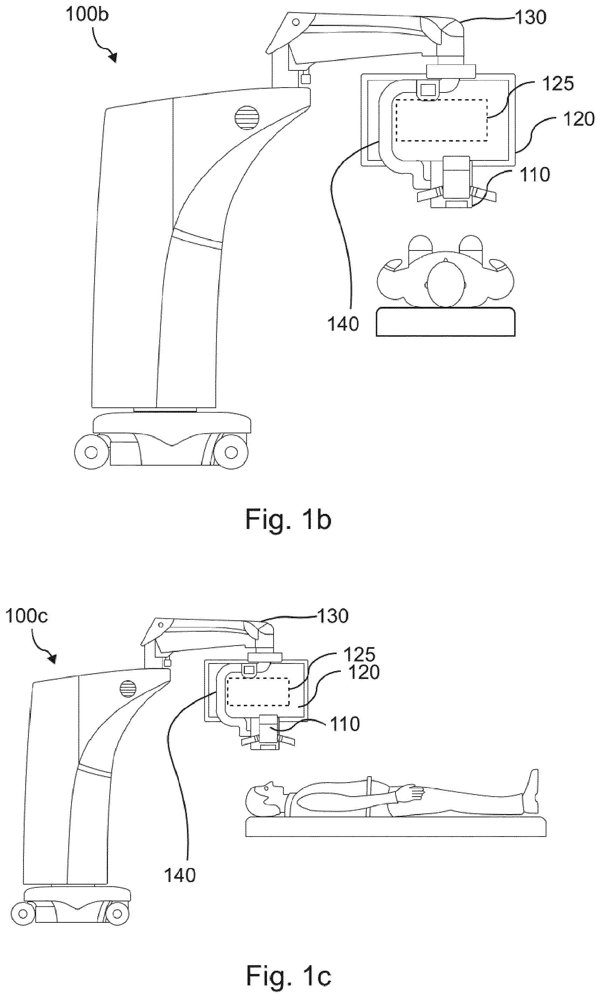

Fig. 1a shows a schematic diagram of an example of a surgical microscope system 100. The surgical microscope system comprises an optics carrier 110 comprising optical components of a microscope of the surgical microscope system. The surgical microscope system comprises a display device 120 for displaying image data recorded by an optical imaging sensor of the surgical microscope system (shown in Figs. 1b to 1e). The surgical microscope system comprises an arm. The arm comprises a first section 130 and a second section 140. The first section is configured to provide a vertical and/or lateral adjustment of the position of the optics carrier. The second section is attached to a suspension point of the first section and to the optics carrier. The second section has a shape that creates a free space around a geometric axis 150 between the suspension point and the optics carrier, such that an optical path between a central area 125 of the display device and a surgeon being located at the opposite side of the optics carrier remains unobstructed by the second section. The optical path intersects the geometric axis between the suspension point and the optics carrier. In Fig. 1a, a base unit of the surgical microscope system is not shown. In general, the first section may be attached to the base unit of the surgical microscope system. Such an attachment is, for example, shown in Figs. 1b to 1e.

In Fig. 1a, merely the arm 130; 140 and the optics carrier of the surgical microscope system are shown. The display device and the base unit of the surgical microscope system are shown in the subsequent Figs., e.g. Figs. 1b to 1e.

Embodiments of the present disclosure relate to a surgical microscope system, i.e. a microscope system to be used during a surgical procedure. In general, a distinction is made between the actual microscope and the overall surgical microscope system, with the surgical microscope system comprising the microscope and various components that are used in conjunction with the optics carrier, e.g. a lighting system, the arm 130; 140, or the display device 120. In such a surgical microscope system, the actual microscope is referred to as the "optics carrier" 110, as it comprises the optical components of the microscope system. In general, a microscope is an optical instrument that is suitable for examining objects that are too small to be examined by the human eye (alone). For example, a microscope may provide an optical magnification of an object. In modern microscopes, the optical magnification is often provided for a camera or an imaging sensor. The optics carrier 110 may further comprise one or more optical magnification components that are used to magnify a view on the sample.

In general, there are various types of optics carriers for surgical microscope system. In the present disclosure, the focus is on optics carriers for use in operating cockpits, where an external display is being used as the main means for the surgeon to view the surgical site. Accordingly, the optics carrier might merely comprise the optical components and the optical imaging sensor, with the image data of the optical imaging sensor being shown on the display device. In this case, the optics carrier may be an optics carrier without an optical (or digital) eyepiece.

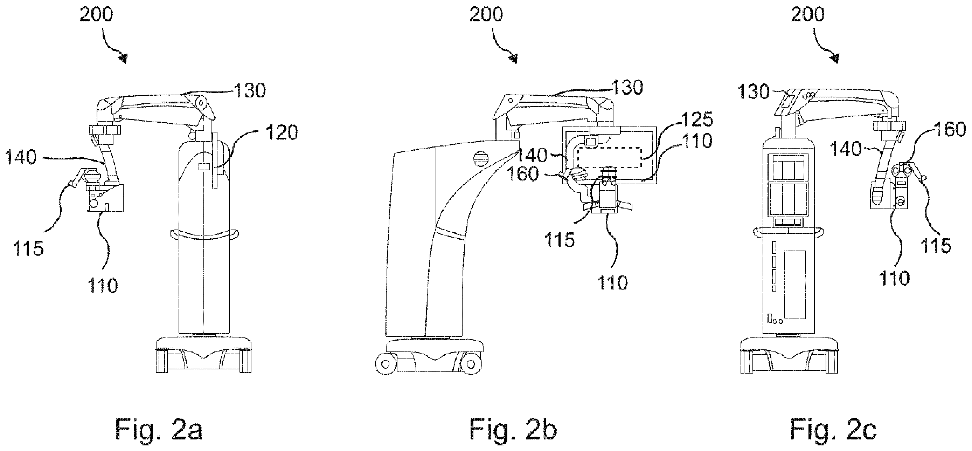

Alternatively, the optics carrier might comprise one or more eyepieces, e.g. an optical or hybrid eyepiece (optical with an optional digital overlay or replacement by a camera image) for use by the main surgeon, and a (digital) eyepiece for an assistant. Such an example is shown in Figs. 2a to 2c.

As has been mentioned above, in the surgical microscope system, an external display is being used as the main means for the surgeon to view the surgical site. This is achieved by recording the surgical site using an optical imaging sensor of the surgical microscope system, which may be included within the optics carrier. In other words, the optics carrier may comprise the optical imaging sensor. For example, the optical imaging sensor of the microscope may comprise or be an APS (Active Pixel Sensor) - or a CCD (Charge-Coupled-Device)-based imaging sensor. For example, in APS-based imaging sensors, light is recorded at each pixel using a photodetector and an active amplifier of the pixel. APS-based imaging sensors are often based on CMOS (Complementary Metal-Oxide-Semiconductor) or S-CMOS (Scientific CMOS) technology. In CCD-based imaging sensors, incoming photons are converted into electron charges at a semiconductor-oxide interface, which are subsequently moved between capacitive bins in the imaging sensor modules by a control circuitry of the optical imaging sensor to perform the imaging.

The image data is subsequently displayed by the display device. For example, the base unit of the surgical microscope system may comprise one or more processors configured to process the image data, and to provide a display signal comprising the processed image data to the display device 120. The display device 120 is configured to display the (processed) image data recorded by the optical imaging sensor of the surgical microscope system.

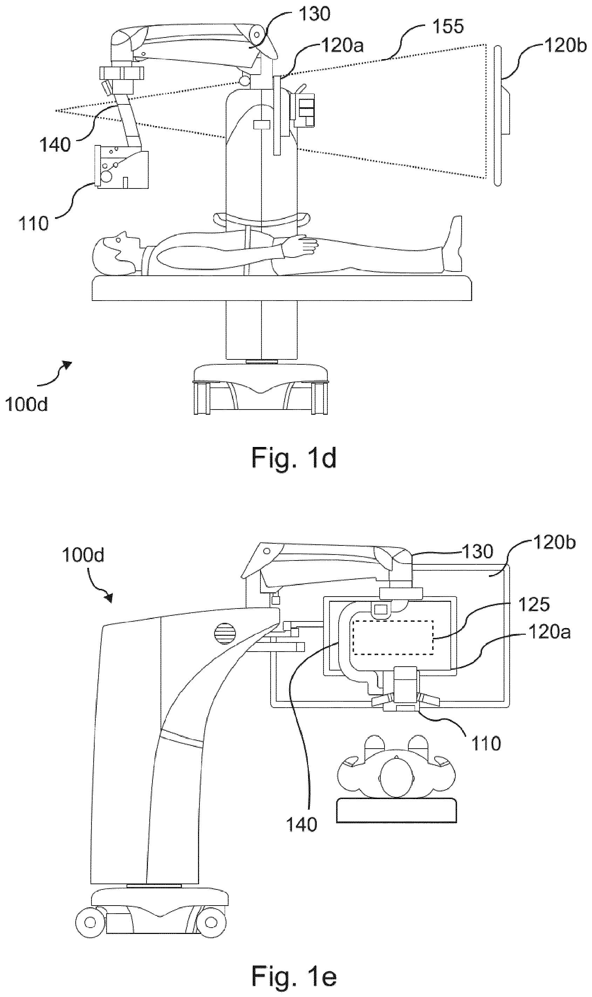

Figs. 1b to 1e show schematic diagrams of examples of the microscope system, where a field of view of a surgeon system towards the display device 120 is shown. In Figs. 1b to 1e, the examples of the surgical microscope system 100 are denoted 100b (Fig. 1b), 100c (Fig. 1c) and 100d (Figs. 1d and 1e). In Figs. 1b to 1e, the display device 120 is shown (with the central portion 125 highlighted by a dashed frame). In general, the display device can be located at several locations (or attached at several locations). However, to allow for ergonomic viewing by the surgeon, the display device is arranged at the opposing side, for the main surgeon, of the optics carrier. In other words, the optics carrier may be located (i.e. arranged) laterally between the display device and a location that is designated for the main surgeon, i.e. a location that enables the use of the handles that are attached to the optics carrier. The patient, and thus the surgical site, may be placed below the optics carrier, as shown in Figs. 1b and 1c.

In general, there are various types of displays that can be included in a surgical microscope system. In particular, the display device being referred to in the present context is an external display, i.e. a display that is not included within the optics carrier or the arm of the surgical microscope system. Moreover, the display device may be arranged independently from the arm of the surgical microscope system, e.g. at a further arm of the surgical microscope system, or at a wall of the operating theatre. In other words, the display device may be a display that is mounted to another arm of the surgical microscope system (e.g. as shown in Fig. 1e for display device 120a) or to a separate stand or at the wall of the operating theatre (e.g. as shown in Fig. 1d / 1e for display device 120b). Accordingly, the surgical microscope system may comprise multiple display devices, which may be arranged, relative to a desired position for the surgeon, in the field of view of the optical path through the geometric axis referenced above.

There are various display technologies that can be used for such displays. For example, the display device may comprise one of a Liquid Crystal Display (LCD) or an Organic Light-Emitting Diode (OLED) display. In some example, a three-dimensional viewing experience may be created. Accordingly, the display device may be a display device for displaying three-dimensional image data. For example, the display device may be configured to provide three-dimensional view on stereoscopic image data provided by the optical imaging sensor (or rather stereoscopic sensors) of the optics carrier. For example, the display device may be configured to output a three-dimensional representation of the image data, e.g. using polarization filters. The surgeons may wear eyeglasses comprising corresponding polarization filters.

The surgical microscope system further comprises the arm with the first section 130 and the second section. In particular, the second section is of relevance, as it is adapted to create the free space around the geometric axis.

The arm comprises the first section, which is configured to provide the vertical and/or lateral adjustment of the position of the optics carrier. For example, as shown in Figs. 1b to 1e, the first section may be attached to a base unit of the surgical microscope system (the base unit comprising the one or more processors) and to the second section of the arm. In other words, the first section may be arranged between the base unit and the second section, and may attach the second section to the base unit. For example, the first section can be moved around with the help of a hydraulic or servo-motor assistance. In general, the first section may be denoted a parallelogram section of the arm of the surgical microscope system. In contrast, the second section may be denoted the optics carrier arm or the optics carrier section.

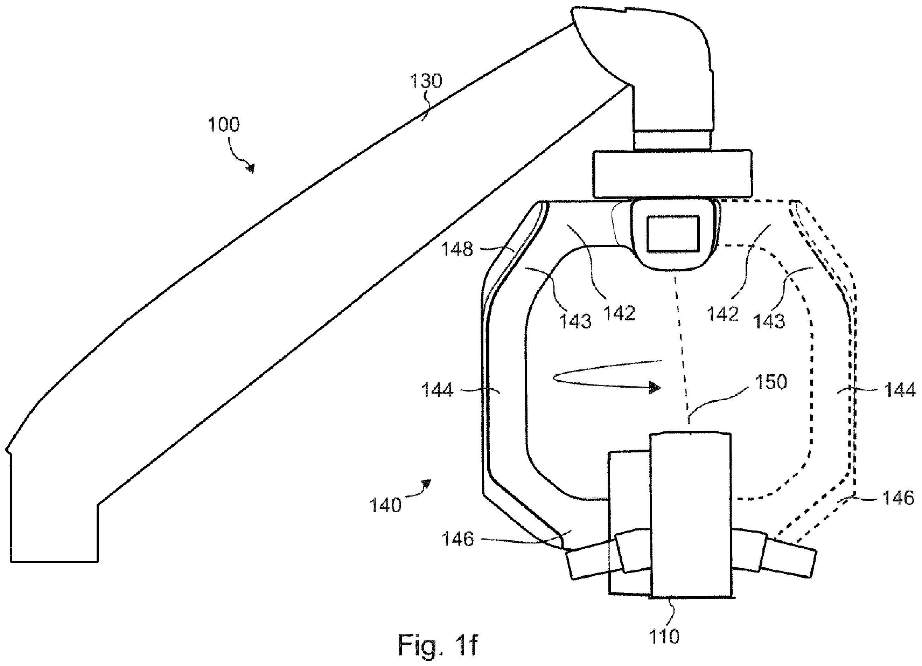

The second section is attached to the first section, i.e. to the suspension point of the first section, and to the optics carrier. The suspension point of the first section may be a portion of the first section that is located (directly) above the optical carrier. In other words, at least a portion of the optics carrier and the suspension point may be intersected by a further geometric axis that runs in parallel to the force of gravity. In some embodiments, the second section is adapted to rotate around the suspension point. For example, the second section may be adapted to rotate around the suspension point, such that a major sub-section of the second section (e.g. the second sub-section, as shown in the following) can be positioned on the left side or on the right side of the optical axis, from the perspective of the surgeon. This is shown in Fig. 1f, for example. Fig. 1f shows a schematic diagram of an example of a surgical microscope system with an arm comprising a first and a second section, where the second section can be rotated (i.e. swiveled) around a suspension point that is part of the first section. In other words, the C-shaped arm (i.e. the second section) may be swiveled around to allow the optical carrier (or optics carrier) to be positioned on either the left (side) or the right (side).

As has been mentioned above, the second section has a shape that creates a free space around the geometric axis 150 between the suspension point and the optics carrier, such that an optical path between a central area 125 of the display device and a surgeon being located at the opposite side of the optics carrier remains unobstructed by the second section. In this context, the term "optical path" might not refer to a single beam between the surgeon and the display device, but rather to an area that is visible in an unobstructed manner by the surgeon, as is shown in Fig. 1d. In Fig. 1d, a triangle 155 is shown that illustrates the optical path between the surgeon and the display device 120a and the display device 120b, with an acute angle being present at a corner of the triangle that faces the surgeon. As can be seen from the illustration, optimally, the entire height of the display devices may be visible in an un-obstructed manner via the optical path. However, this might not be a pre-requisite, as the most important information is usually displayed within the central area of the display device. For clarification, the central area is centered around a center point of the display device. The central area may occupy at least 20% (or at least 30%, at least 40%, at least 50%, at least 60%, at least 70%, at least 80%) of an overall area of the display device. For example, the central area may occupy at least 40% (or at least 50%, at least 60%, at least 70%, at least 80%, at least 90%) of the overall lateral width of a screen the display device and/or at least 40% (or at least 50%, at least 60%, at least 70%, at least 80%, at least 90%) of the overall vertical height of the screen the display device.

The optical path intersects the geometric axis between the suspension point and the optics carrier. In other words, at least a portion of the optical path intersects the geometric axis between the suspension point. Moreover, the geometric axis may be at the center of the optical path, i.e. the surgeon may look through the second section towards the central area through the geometric axis. The display device may be arranged such that the surgeon looks through the free space created by the shape of the second section towards the display device. In other words, a central beam of the optical path (between the surgeon and the display device) may intersect the geometric axis.

This may, for example, be achieved with various shapes and forms. For example, the second section may be shaped like a lying "O", with a recess of the lying "O" creating the free space. Preferably, however, the second section may be shaped like a "C" or like a lying "V". In other words, the second section is substantially shaped like the letter "C" or like the letter "V" turned on its side. In some cases, the shape may also be likened to the shape of a square bracket or a round bracket.

By performing a redesign of the optics carrier arm into an extended "C-shaped or V-shaped" structure, a wider field of view over the microscope stack is enabled. This allows an unobstructed true frontal viewing of surgical monitors, leading to better hand-eye coordination and a more natural working position, without the need of tilting the head off-centre. In addition, this allows the surgeon to better observe the clinical setup and workflow in front of him/ her. This may improve their workflow efficiency from an unobstructed visualisation of the tools needed and easy identification of data readouts from critical surgical instruments. Figs. 1b and 1c illustrate the unobstructed true frontal viewing of surgical monitors for both superior and temporal operating positions. Figs. 1d and 1e illustrate unobstructed frontal viewing of both the stand monitor 120a and larger screen monitors 120b positioned further away.

In more specific terms, the shape of the second section may comprise a first sub-section 142 that extends laterally outwards from the suspension point, a second sub-section 144 that leads downwards from the first sub-section, and a third sub-section 146 that subsequently returns inwards from the second sub-section towards the optics carrier.

The first sub-section 142 may extend laterally outwards from the suspension point. However, the first sub-section might not be directly attached to the suspension point. Instead, the second section may comprise a further sub-section that is arranged between the first sub-section and the suspension point. In Fig. 1a, this further sub-section is shown as a wide rectangle. The first sub-section 142 is attached to said rectangle and extends laterally outwards from the suspension point. In this context, the term "extends laterally outwards" indicates two properties of the first sub-section - that the first section leads away (i.e. outwards) from a lateral position of the suspension point, and that it comprises a substantial lateral extent (e.g. that the first sub-section extends outwards for at least 15 cm), i.e. a lateral extent that causes a transition between the first and second sub-section to be outside the geometric axis between the suspension point and the optics carrier. In other words, the first sub-section may lead the second section away from the geometric axis between the suspension point and the optics carrier.

The second sub-section 144 leads downwards from the first sub-section. In other words, a first end of the second sub-section 144 that is attached to the first sub-section may be located above a second end of the second sub-section 144 that is attached to the third sub-section. Furthermore, the second sub-section may be attached to both the first and the third sub-section (e.g. via transitional sections), and be arranged in-between the first and third sub-section.

The second sub-section leads downwards for at least 20 cm (or at least 25 cm, at least 30 cm, at least 40 cm). In other words, a first end of the second sub-section 144 that is attached to the first sub-section is ocated at least 20 cm (or at least 25 cm, at least 30 cm, at least 40 cm) above a second end of the second sub-section 144 that is attached to the third sub-section. For example, the second sub-section may comprise a portion that leads straight downwards (i.e. (substantially) in parallel to the force of gravity). The portion may occupy at least 40% (or at least 50%, at least 60%, at least 70%, at least 80%) of a vertical height of the second sub-section. In other words, the portion may have a vertical height of at least 10 cm (or at least 12 cm, at least 15 cm, at least 20 cm, at least 25 cm, at least 30 cm, at least 35 cm).

The third sub-section 146 may subsequently return inwards from the second sub-section towards the optics carrier. In this context, the term "inwards" indicates that the third sub-section leads from the second sub-section towards the geometric axis between the suspension point and the optics carrier. In general, the third section may be on the same vertical level as the optics carrier. Accordingly, the third sub-section may lead laterally towards the optics carrier, on the same level as the optics carrier. For example, the optics carrier may be attached to the third section, e.g. via an additional subsection, or via an interposer.

In some examples, the second section further comprises transitional sub-sections that are arranged between the respective sub-sections and that provide a gradual transition between the respective sections. In the case of the transition between the first and second sub-section, the transition may be slanted, so that the second section does not collide with the first section in a parked position of the arm of the surgical microscope system. For example, the second section may comprise curved edges to allow the optics carrier to be brought into a parked position without colliding with the microscope arm. In other words, the transition 143 between the first and second sub-section may be slanted, such that, in a parked position of the first and second section of the arm of the surgical microscope system, a pre-defined distance 143a remains between the first section and the slanted transition between the first and second sub-section of the second section. This distance 143a is shown in Fig. 1a. For example, the transition between the first and second sub-section may be slanted such, that a tilt of the transition approximates a tilt of the first section in a parked position of the surgical microscope system. For example, the pre-defined distance may be at least 2 cm (or 3 cm, or 4 cm) and/or at most 12 cm (or 10 cm, or 8 cm, or 6 cm).

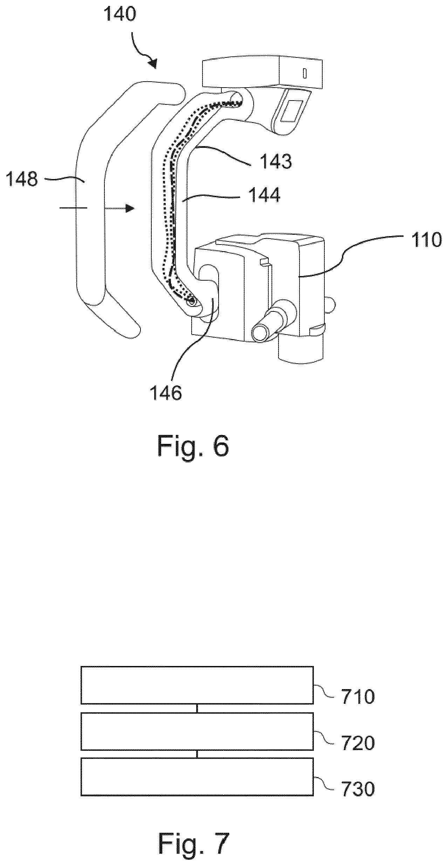

As has been mentioned above, the optics carrier may comprise optical imaging devices for generating image data that is to be displayed on the display device, and various other components. These components may require an electrical and/or data connection to the base unit of the surgical microscope system. Additionally, the optics carrier may comprise an illumination system of the surgical microscope system, or a fiber conductor may be used to conduct the light to the optics carrier, to be provided to the surgical site via the optics carrier. All of these features may require a connection between the base unit of the surgical microscope system and the optical carrier. Therefore, the second section (as well as the first section) may comprise a cable duct. Additionally, the second section may comprise a removable cover 148 for accessing cables within the cable duct. For example, the removable cover may be arranged at a side of the second section facing away from the geometric axis between the suspension point and the optics carrier. A more detailed view of such a cable duct and cover is shown in Fig. 6.

In the context of the present disclosure, the term "lateral" or "horizontal" is defined in parallel to a floor level of a room the surgical microscope system is being used in, which may be perpendicular to the force of gravity. The term "vertical" is defined perpendicular to the floor level, and thus in parallel to the force of gravity. Correspondingly, the terms "above" and "below" may be defined with respect to the vertical direction, i.e. if a first component is located above a second component it means that at least a bulk (or the entirety) of the first component is located vertically above the second component (i.e. vertically further away from the floor level of the room the surgical microscope system is located in), if the first component is located below the second component it means that at least the bulk (or the entirety) of the first component is located vertically below the second component (i.e. vertically closer to the floor level of the room the surgical microscope system is located in).

More details and aspects of the surgical microscope system are mentioned in connection with the proposed concept or one or more examples described above or below (e.g. Fig. 2a to 7). The surgical microscope system may comprise one or more additional optional features corresponding to one or more aspects of the proposed concept or one or more examples described above or below.

Figs. 2a to 2c show schematic diagrams of an example of a surgical microscope system 200 with two eyepieces 115; 160 from different perspectives. In general, the surgical microscope system of Figs. 2a and 2b may be implemented similar to the surgical microscope system 100 (100; 100b; 100c; 100d) of Figs. 1a to 1e. However, in contrast to the examples shown in connection with Figs. 1a to 1e, the surgical microscope system 200 of Figs. 2a to 1c comprises a first eyepiece 115 (for the main surgeon) and a second eyepiece 160 (for an assistant). In other words, the optics carrier may comprise, at a side of the optics carrier the second section is attached at, a digital eyepiece 160 and/or, at a side of the optics carrier facing the surgeon, an optical or hybrid eyepiece 115. In general, any combination may be possible, e.g. a surgical microscope system with the first eyepiece 115 but without the second eyepiece 160, a surgical microscope system with the second eyepiece 160 but without the first eyepiece 115, a surgical microscope system without any eyepieces, or a surgical microscope system with both eyepieces 115; 160. For example, the first eyepiece may be an optical eyepiece or a hybrid optical and digital eyepiece, and the second eyepiece may be a digital eyepiece. In this context, the term "digital eyepiece" indicates that the view on the sample through the eyepiece is generated exclusively by a display that is integrated within, or connected to, the eyepiece. The term "hybrid optical and digital eyepiece" indicates that the eyepiece provides both a purely optical and a view that is generated by a display, and optionally a view with a digital overlay over the optical view. In general, the eyepieces may also be denoted binoculars.

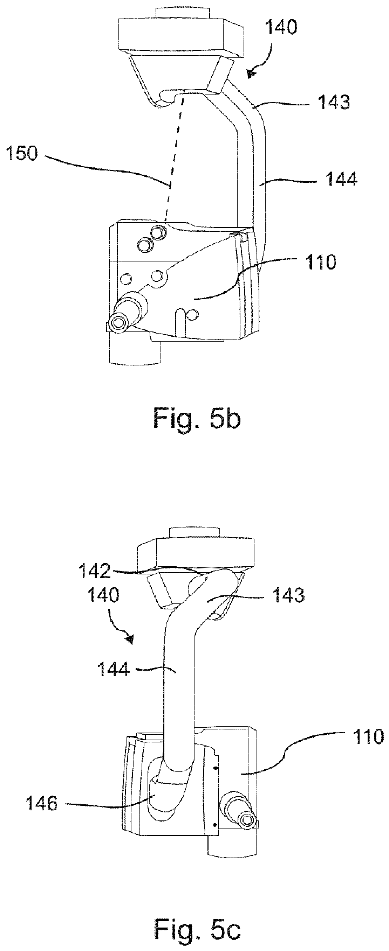

Figs. 2a to 2c shows a curved optics carrier arm 140 accommodating the main binoculars (eyepiece) and side assistant binoculars (eyepiece). Accordingly, to allow for the placement of the second eyepiece 160, the second section may be partially slanted towards the display device. In other words, the shape of the second section may be such, that a first lateral distance between a sub-section of the second section that is attached to the optics carrier and the display device is smaller than a lateral distance between a sub-section of the second section that is attached to the first section and the display device. In other words, the lower portion of the second section may be slanted towards the display device. Such an arrangement is shown in Figs. 2a to 2c, where the entire second sub-section of the second section is tilted. Another implementation is shown in Fig. 5c, where only a portion of the second sub-section is tilted, and a main portion of the second subsection leads straight downwards.

More details and aspects of the surgical microscope system are mentioned in connection with the proposed concept or one or more examples described above or below (e.g. Fig. 1a too 1e, 3a to 7). The surgical microscope system may comprise one or more additional optional features corresponding to one or more aspects of the proposed concept or one or more examples described above or below.

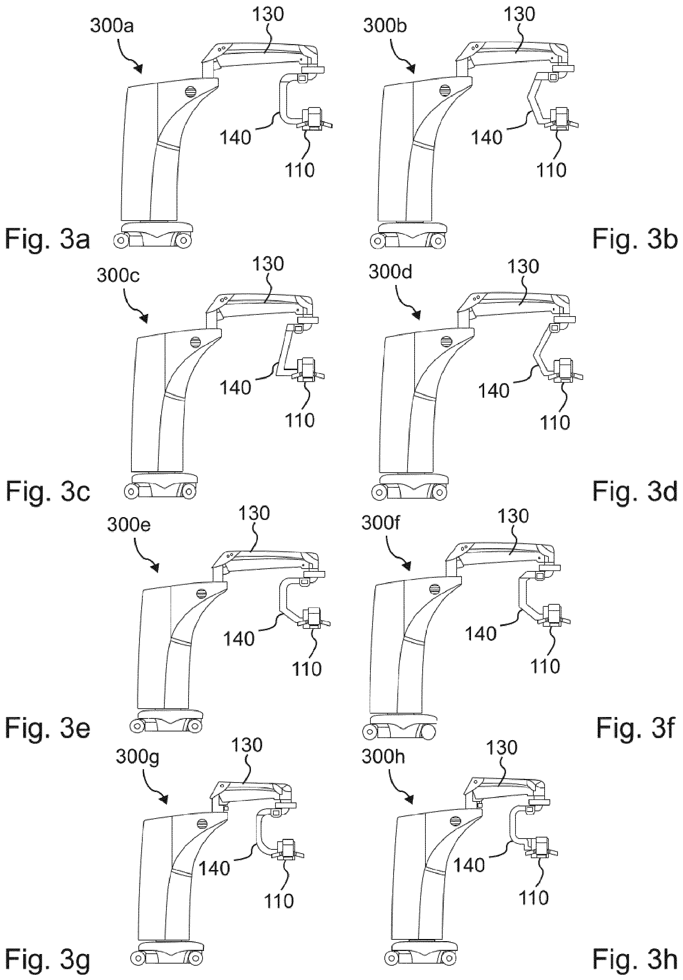

Figs. 3a to 3h show different shapes of portions of a second section 130 of an arm of a surgical microscope system 300a-300h. For example, the different shapes may be used to implement the second section of the arm of the surgical microscope systems shown in Figs. 1a to 2c, 4 to 6. Figs. 3a to 3h may show a "C-shaped or V-shaped" optics carrier arm that enables a wider field of view over the microscope stack.

In Fig. 3a "C-shaped" second section 140 is shown, with a first, second and third subsection that extend substantially laterally (first and third-subsection) or vertically (second subsection), with only a small transition between the subsections. In Fig. 3b, a shallow "V-shaped" second section is shown, with the first and third sub-section that extend substantially laterally, and a second sub-section that has the shape of a shallow, lying "V". In Fig. 3c, a "C-shaped" second section is shown that resembles a cursive C, with the first and third sub-section that extend substantially laterally, and a second sub-section that leads downwards and outwards at the same time. The second section of Fig. 3d is similar to the second section of Fig. 3b, with the second sub-section having a more pronounced "V-shape". In Figs. 3e and 3f, the first subsection extends substantially laterally outwards, and the second sub-section leads straight downwards, with the third sub-section leading downwards and inwards at the same time. The second section of Fig. 3g is similar to the second section of Fig. 3a, with the second sub-section having a rounded shape that leads laterally outwards before returning laterally inwards as the second sub-section meets the third sub-section. The second section of Fig. 3h is also similar to the second section of Fig. 3a, with the second section comprising a fourth subsection that is arranged between the third sub-section and the optics carrier, and that leads both downwards and inwards in order to lower the lateral position of the optics carrier 110 above ground.

More details and aspects of the surgical microscope system are mentioned in connection with the proposed concept or one or more examples described above or below (e.g. Fig. 1a to 2c, 4 to 7). The surgical microscope system may comprise one or more additional optional features corresponding to one or more aspects of the proposed concept or one or more examples described above or below.

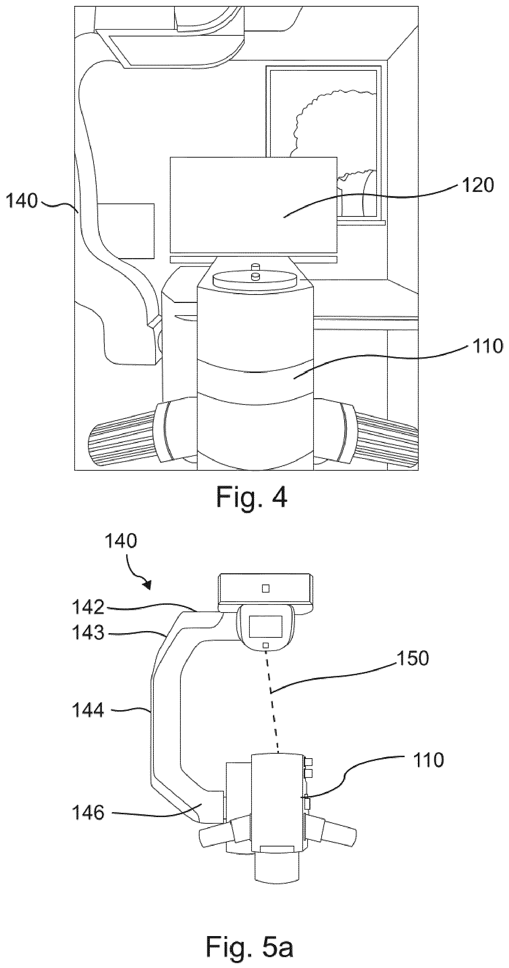

Fig. 4 illustrates a field of view of a surgeon through a second section 140 of a surgical microscope system is shown according to an example. The view is shown according to a functional model, showing an unobstructed, wide front field of view by the surgeon

More details and aspects of the surgical microscope system are mentioned in connection with the proposed concept or one or more examples described above or below (e.g. Fig. 1a to 3h, 5a to 7). The surgical microscope system may comprise one or more additional optional features corresponding to one or more aspects of the proposed concept or one or more examples described above or below.

Figs. 5a to 5c show schematic diagrams of a second section of an arm 140 of a surgical microscope system 500 from different perspectives. For example, the surgical microscope system 500 of Figs. 5a to 5c may be implemented similar to the surgical microscope systems 100-300h of Figs. 1a to 3h. Figs. 5a to 5c show front and side concept views of the new optics carrier arm. For example, Figs. 5a to 5c may show a more detailed view of the second section used in Figs. 1a to 2c. As is evident from Fig. 5c, a vertical height of the third sub-section may be at least 20% larger than a lateral width of the third sub-section. This may improve the stability of the attachment of the optics carrier 110 at the second section.

More details and aspects of the surgical microscope system are mentioned in connection with the proposed concept or one or more examples described above or below (e.g. Fig. 1a to 4, 6 to 7). The surgical microscope system may comprise one or more additional optional features corresponding to one or more aspects of the proposed concept or one or more examples described above or below.

In Fig. 6, a cable duct being integrated within the second section is shown. Fig. 6 shows a schematic diagram of a second section of an arm of a surgical microscope, with a cable duct and with a removable cover 148. Fig. 6 may illustrate cabling access to internal wiring.

More details and aspects of the surgical microscope system are mentioned in connection with the proposed concept or one or more examples described above or below (e.g. Fig. 1a to 5c, 7). The surgical microscope system may comprise one or more additional optional features corresponding to one or more aspects of the proposed concept or one or more examples described above or below.

Fig. 7 shows a flow chart of a method for a surgical microscope system. For example, the method may be suitable for one of the surgical microscope system introduced in connection with Figs. 1a to 6. The method comprises providing 710 an optics carrier of the surgical microscope system, the optics carrier comprising optical components of a microscope of the surgical microscope system. The method comprises providing 720 a display device of the surgical microscope system, the display device being suitable for displaying image data recorded by an optical imaging sensor of the surgical microscope system. The method comprises providing 730 an arm of the surgical microscope system, the arm comprising a first section and a second section. The first section is configured to provide a vertical and/or lateral adjustment of the position of the optics carrier. The second section is attached to a suspension point of the first section and to the optics carrier. The arm is provided with the second section having a shape that creates a free space around a geometric axis between the suspension point and the optics carrier, such that an optical path between a central area of the display device and a surgeon being located at the opposite side of the optics carrier remains unobstructed by the second section, the optical path intersecting the geometric axis between the suspension point and the optics carrier. The display device is provided at a side of the optics carrier that is opposite a desired position of the surgeon.

More details and aspects of the method for the surgical microscope system are mentioned in connection with the proposed concept or one or more examples described above or below (e.g. Fig. 1a to 6). The method may comprise one or more additional optional features corresponding to one or more aspects of the proposed concept or one or more examples described above or below.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

The aspects and features described in relation to a particular one of the previous examples may also be combined with one or more of the further examples to replace an identical or similar feature of that further example or to additionally introduce the features into the further example.

The following claims are hereby incorporated in the detailed description, wherein each claim may stand on its own as a separate example. It should also be noted that although in the claims a dependent claim refers to a particular combination with one or more other claims, other examples may also include a combination of the dependent claim with the subject matter of any other dependent or independent claim. Such combinations are hereby explicitly proposed, unless it is stated in the individual case that a particular combination is not intended. Furthermore, features of a claim should also be included for any other independent claim, even if that claim is not directly defined as dependent on that other independent claim.

### List of reference Signs

- 100, 100b-d: Surgical microscope systems
- 110: Optics carrier
- 115: Eyepiece
- 120, 120a/b: Display devices
- 125: Central area
- 130: First section of an arm of the surgical microscope system
- 140: Second section of an arm of the surgical microscope system
- 142: First sub-section
- 143: Transition between first sub-section and second sub-section
- 143a: Distance between transition and first section
- 144: Second sub-section
- 146: Third sub-section
- 148: Removable cover
- 150: Geometric axis
- 155: Triangle
- 160: Eyepiece
- 200: Surgical microscope system
- 300a-300h: Surgical microscope systems
- 710: Providing an optics carrier
- 720: Providing a display device
- 730: Providing an arm

## Claims

1. A surgical microscope system (100) comprising:
an optics carrier (110) comprising optical components of a microscope of the surgical microscope system;
a display device (120) for displaying image data recorded by an optical imaging sensor of the surgical microscope system; and
an arm, the arm comprising a first section (130) and a second section (140), the first section being configured to provide a vertical and/or lateral adjustment of the position of the optics carrier, the second section being attached to a suspension point of the first section and to the optics carrier,
the second section having a shape that creates a free space around a geometric axis (150) between the suspension point and the optics carrier, such that an optical path between a central area (125) of the display device and a surgeon being located at the opposite side of the optics carrier remains unobstructed by the second section, the optical path intersecting the geometric axis between the suspension point and the optics carrier, wherein
the shape of the second section comprises a first sub-section (142) that extends laterally outwards from the suspension point, a second sub-section (144) that leads downwards from the first sub-section, and a third sub-section (146) that subsequently returns inwards from the second sub-section towards the optics carrier and **characterised in that** the second sub-section (144) leads downwards for at least 20 cm.

2. The surgical microscope system according to claim 1, wherein the central area is centered around a center point of the display device,
and/or wherein the central area occupies at least 20% of an overall area of the display device.

3. The surgical microscope system according to any one of the preceding claims, wherein the second section is substantially shaped like the letter "C".

4. The surgical microscope system according to any one of the preceding claims, wherein the first sub-section extends outwards for at least 15 cm.

5. The surgical microscope system according to any one of the preceding claims, wherein the second sub-section comprises a portion that leads straight downwards, the portion occupying at least 40% of a vertical height of the second sub-section.

6. The surgical microscope system according to any one of the preceding claims, wherein a transition (143) between the first and second sub-section is slanted, such that, in a parked position of the first and second section of the arm of the surgical microscope system, a pre-defined distance (143a) remains between the first section and the slanted transition between the first and second sub-section of the second section.

7. The surgical microscope system according to any one of the preceding claims, wherein a vertical height of the third sub-section is at least 20% larger than a lateral width of the third sub-section.

8. The surgical microscope system according to any one of the preceding claims, wherein the display device is a display device for displaying three-dimensional image data.

9. The surgical microscope system according to any one of the preceding claims, wherein the optics carrier is an optics carrier without an optical eyepiece,
and/or wherein the second section comprises a cable duct and a removable cover (148) for accessing cables within the cable duct.

10. The surgical microscope system according to any one of the claims, wherein the shape of the second section is such, that a first lateral distance between a sub-section of the second section that is attached to the optics carrier and the display device is smaller than a lateral distance between a sub-section of the second section that is attached to the first section and the display device.

11. The surgical microscope system according to claim 10, wherein the optics carrier comprises, at a side of the optics carrier the second section is attached at, a digital eyepiece (160).

12. The surgical microscope system according to any one of the preceding claims, wherein the second section is adapted to rotate around the suspension point.

13. A method for a surgical microscope system, the method comprising:
providing (710) an optics carrier of the surgical microscope system, the optics carrier comprising optical components of a microscope of the surgical microscope system;
providing (720) a display device of the surgical microscope system, the display device being suitable for displaying image data recorded by an optical imaging sensor of the surgical microscope system; and
providing (730) an arm of the surgical microscope system, the arm comprising a first section and a second section, the first section being configured to provide a vertical and/or lateral adjustment of the position of the optics carrier, the second section being attached to a suspension point of the first section and to the optics carrier,
wherein the arm is provided with the second section having a shape that creates a free space around a geometric axis between the suspension point and the optics carrier, such that an optical path between a central area of the display device and a surgeon being located at the opposite side of the optics carrier remains unobstructed by the second section, the optical path intersecting the geometric axis between the suspension point and the optics carrier, the display device being provided at a side of the optics carrier that is opposite a desired position of the surgeon, wherein
the shape of the second section comprises a first sub-section (142) that extends laterally outwards from the suspension point, a second sub-section (144) that leads downwards from the first sub-section, and a third sub-section (146) that subsequently returns inwards from the second sub-section towards the optics carrier and **characterised in that** the second sub-section (144) leads downwards for at least 20 cm.

## Patentansprüche

1. Chirurgisches Mikroskopsystem (100), umfassend:
einen Optikträger (110), umfassend optische Komponenten eines Mikroskops des chirurgischen Mikroskopsystems;
eine Anzeigevorrichtung (120) zum Anzeigen von Bilddaten, die durch einen optischen Bildsensor des chirurgischen Mikroskopsystems aufgezeichnet werden; und
einen Arm, wobei der Arm einen ersten Abschnitt (130) und einen zweiten Abschnitt (140) umfasst, wobei der erste Abschnitt ausgebildet ist, eine vertikale und/oder seitliche Verstellung der Position des Optikträgers zu ermöglichen, wobei der zweite Abschnitt an einem Aufhängepunkt des ersten Abschnitts und an dem Optikträger angebracht ist,
wobei der zweite Abschnitt eine Form aufweist, die einen Freiraum um eine geometrische Achse (150) zwischen dem Aufhängepunkt und dem Optikträger schafft, derart, dass ein optischer Pfad zwischen einem zentralen Bereich (125) der Anzeigevorrichtung und einem Chirurgen, der sich auf der gegenüberliegenden Seite des Optikträgers befindet, durch den zweiten Abschnitt frei bleibt, wobei der optische Pfad die geometrische Achse zwischen dem Aufhängepunkt und dem Optikträger schneidet, wobei
die Form des zweiten Abschnitts einen ersten Unterabschnitt (142) umfasst, der sich seitlich nach außen von dem Aufhängepunkt erstreckt, einen zweiten Unterabschnitt (144), der von dem ersten Unterabschnitt nach unten führt, und einen dritten Unterabschnitt (146), der anschließend von dem zweiten Unterabschnitt wieder nach innen in Richtung des Optikträgers verläuft, und **dadurch gekennzeichnet, dass** der zweite Unterabschnitt (144) für mindestens 20 cm nach unten führt.

2. Chirurgisches Mikroskopsystem nach Anspruch 1, wobei der zentrale Bereich um einen Mittelpunkt der Anzeigevorrichtung zentriert ist,
und/oder wobei der zentrale Bereich mindestens 20 % einer Gesamtfläche der Anzeigevorrichtung einnimmt.

3. Chirurgisches Mikroskopsystem nach einem der vorstehenden Ansprüche, wobei der zweite Abschnitt im Wesentlichen wie der Buchstabe "C" geformt ist.

4. Chirurgisches Mikroskopsystem nach einem der vorstehenden Ansprüche, wobei sich der erste Unterabschnitt für mindestens 15 cm nach außen erstreckt.

5. Chirurgisches Mikroskopsystem nach einem der vorstehenden Ansprüche, wobei der zweite Unterabschnitt einen Abschnitt umfasst, der gerade nach unten führt, wobei der Abschnitt mindestens 40 % einer vertikalen Höhe des zweiten Unterabschnitts einnimmt.

6. Chirurgisches Mikroskopsystem nach einem der vorstehenden Ansprüche, wobei ein Übergang (143) zwischen dem ersten und dem zweiten Unterabschnitt schräg ist, derart, dass in einer Parkposition des ersten und des zweiten Abschnitts des Arms des chirurgischen Mikroskopsystems ein vordefinierter Abstand (143a) zwischen dem ersten Abschnitt und dem schrägen Übergang zwischen dem ersten und dem zweiten Unterabschnitt des zweiten Abschnitts verbleibt.

7. Chirurgisches Mikroskopsystem nach einem der vorstehenden Ansprüche, wobei eine vertikale Höhe des dritten Unterabschnitts mindestens 20 % größer als eine seitliche Breite des dritten Unterabschnitts ist.

8. Chirurgisches Mikroskopsystem nach einem der vorstehenden Ansprüche, wobei die Anzeigevorrichtung eine Anzeigevorrichtung zum Anzeigen von dreidimensionalen Bilddaten ist.

9. Chirurgisches Mikroskopsystem nach einem der vorstehenden Ansprüche, wobei der Optikträger ein Optikträger ohne ein optisches Okular ist,
und/oder wobei der zweite Abschnitt einen Kabelkanal und eine abnehmbare Abdeckung (148) zum Zugreifen auf Kabel innerhalb des Kabelkanals umfasst.

10. Chirurgisches Mikroskopsystem nach einem der Ansprüche, wobei die Form des zweiten Abschnitts derart ist, dass ein erster seitlicher Abstand zwischen einem Unterabschnitt des zweiten Abschnitts, der an dem Optikträger angebracht ist, und der Anzeigevorrichtung kleiner ist als ein seitlicher Abstand zwischen einem Unterabschnitt des zweiten Abschnitts, der an dem ersten Abschnitt angebracht ist, und der Anzeigevorrichtung.

11. Chirurgisches Mikroskopsystem nach Anspruch 10, wobei der Optikträger an einer Seite des Optikträgers, an der der zweite Abschnitt angebracht ist, ein digitales Okular (160) umfasst.

12. Chirurgisches Mikroskopsystem nach einem der vorstehenden Ansprüche, wobei der zweite Abschnitt zum Rotieren um den Aufhängepunkt angepasst ist.

13. Verfahren für ein chirurgisches Mikroskopsystem, das Verfahren umfassend:
Bereitstellen (710) eines Optikträgers des chirurgischen Mikroskopsystems, wobei der Optikträger optische Komponenten eines Mikroskops des chirurgischen Mikroskopsystems umfasst; Bereitstellen (720) einer Anzeigevorrichtung des chirurgischen Mikroskopsystems, wobei die Anzeigevorrichtung zum Anzeigen von Bilddaten geeignet ist, die durch einen optischen Bildsensor des chirurgischen Mikroskopsystems aufgezeichnet werden; und
Bereitstellen (730) eines Arms des chirurgischen Mikroskopsystems, wobei der Arm einen ersten Abschnitt und einen zweiten Abschnitt umfasst, wobei der erste Abschnitt ausgebildet ist, eine vertikale und/oder seitliche Verstellung der Position des Optikträgers bereitzustellen, wobei der zweite Abschnitt an einem Aufhängepunkt des ersten Abschnitts und an dem Optikträger angebracht ist,
wobei der Arm mit dem zweiten Abschnitt bereitgestellt wird, der eine Form aufweist, die einen Freiraum um eine geometrische Achse zwischen dem Aufhängepunkt und dem Optikträger schafft, derart, dass ein optischer Pfad zwischen einem zentralen Bereich der Anzeigevorrichtung und einem Chirurgen, der sich auf der gegenüberliegenden Seite des Optikträgers befindet, durch den zweiten Abschnitt frei bleibt, wobei der optische Pfad die geometrische Achse zwischen dem Aufhängepunkt und dem Optikträger schneidet, wobei die Anzeigevorrichtung auf einer Seite des Optikträgers bereitgestellt wird, die einer gewünschten Position des Chirurgen gegenüberliegt, wobei
die Form des zweiten Abschnitts einen ersten Unterabschnitt (142) umfasst, der sich seitlich nach außen von dem Aufhängepunkt erstreckt, einen zweiten Unterabschnitt (144), der von dem ersten Unterabschnitt nach unten führt, und einen dritten Unterabschnitt (146), der anschließend von dem zweiten Unterabschnitt wieder nach innen in Richtung des Optikträgers verläuft, und **dadurch gekennzeichnet, dass** der zweite Unterabschnitt (144) für mindestens 20 cm nach unten führt.

## Revendications

1. Système de microscope chirurgical (100) comprenant :
un support optique (110) comprenant des composants optiques d'un microscope du système de microscope chirurgical ;
un dispositif d'affichage (120) pour afficher des données d'image enregistrées par un capteur d'imagerie optique du système de microscope chirurgical ; et
un bras, le bras comprenant une première section (130) et une deuxième section (140), la première section étant configurée pour fournir un ajustement vertical et/ou latéral de la position du support optique, la deuxième section étant fixée à un point de suspension de la première section et au support optique,
la deuxième section présentant une forme qui crée un espace libre autour d'un axe géométrique (150) entre le point de suspension et le support optique, de telle sorte qu'un trajet optique entre une zone centrale (125) du dispositif d'affichage et un chirurgien situé sur le côté opposé du support optique reste non obstrué par la deuxième section, le trajet optique intersectant l'axe géométrique entre le point de suspension et le support optique, système de microscope chirurgical, dans lequel
la forme de la deuxième section comprend une première sous-section (142) qui s'étend latéralement vers l'extérieur depuis le point de suspension, une deuxième sous-section (144) qui s'étend vers le bas depuis la première sous-section, et une troisième sous-section (146) qui revient vers l'intérieur depuis la deuxième sous-section vers le support optique, le système de microscope chirurgical étant **caractérisé en ce que** la deuxième sous-section (144) s'étend vers le bas sur au moins 20 cm.

2. Système de microscope chirurgical selon la revendication 1, dans lequel la zone centrale est centrée autour d'un point central du dispositif d'affichage,
et/ou dans lequel la zone centrale occupe au moins 20 % d'une surface totale du dispositif d'affichage.

3. Système de microscope chirurgical selon l'une quelconque des revendications précédentes, dans lequel la deuxième section présente sensiblement la forme de la lettre « C ».

4. Système de microscope chirurgical selon l'une quelconque des revendications précédentes, dans lequel la première sous-section s'étend vers l'extérieur sur au moins 15 cm.

5. Système de microscope chirurgical selon l'une quelconque des revendications précédentes, dans lequel la deuxième sous-section comprend une portion qui s'étend en ligne droite vers le bas, la portion occupant au moins 40 % d'une hauteur verticale de la deuxième sous-section.

6. Système de microscope chirurgical selon l'une quelconque des revendications précédentes, dans lequel une transition (143) entre la première et la deuxième sous-section est inclinée, de telle sorte que, dans une position de stationnement de la première et de la deuxième section du bras du système de microscope chirurgical, une distance prédéfinie (143a) demeure entre la première section et la transition inclinée entre la première et la deuxième sous-section de la deuxième section.

7. Système de microscope chirurgical selon l'une quelconque des revendications précédentes, dans lequel une hauteur verticale de la troisième sous-section est au moins 20 % plus grande qu'une largeur latérale de la troisième sous-section.

8. Système de microscope chirurgical selon l'une quelconque des revendications précédentes, dans lequel le dispositif d'affichage est un dispositif d'affichage pour afficher des données d'image tridimensionnelle.

9. Système de microscope chirurgical selon l'une quelconque des revendications précédentes, dans lequel le support optique est un support optique sans oculaire optique, et/ou dans lequel la deuxième section comprend une goulotte de câbles et un couvercle amovible (148) pour accéder à des câbles à l'intérieur de la goulotte de câbles.

10. Système de microscope chirurgical selon l'une quelconque des revendications, dans lequel la forme de la deuxième section est telle qu'une première distance latérale entre une sous-section de la deuxième section qui est fixée au support optique et le dispositif d'affichage est plus petite qu'une distance latérale entre une sous-section de la deuxième section qui est fixée à la première section et le dispositif d'affichage.

11. Système de microscope chirurgical selon la revendication 10, dans lequel le support optique comprend, sur un côté du support optique auquel la deuxième section est fixée, un oculaire numérique (160).

12. Système de microscope chirurgical selon l'une quelconque des revendications précédentes, dans lequel la deuxième section est adaptée à tourner autour du point de suspension.

13. Procédé pour un système de microscope chirurgical, le procédé comprenant :
la fourniture (710) d'un support optique du système de microscope chirurgical, le support optique comprenant des composants optiques d'un microscope du système de microscope chirurgical ; la fourniture (720) d'un dispositif d'affichage du système de microscope chirurgical, le dispositif d'affichage étant approprié pour afficher des données d'image enregistrées par un capteur d'imagerie optique du système de microscope chirurgical ; et
la fourniture (730) d'un bras du système de microscope chirurgical, le bras comprenant une première section et une deuxième section, la première section étant configurée pour fournir un ajustement vertical et/ou latéral de la position du support optique, la deuxième section étant fixée à un point de suspension de la première section et au support optique,
dans lequel le bras est fourni avec la deuxième section présentant une forme qui crée un espace libre autour d'un axe géométrique entre le point de suspension et le support optique, de telle sorte qu'un trajet optique entre une zone centrale du dispositif d'affichage et un chirurgien situé sur le côté opposé du support optique reste non obstrué par la deuxième section, le trajet optique intersectant l'axe géométrique entre le point de suspension et le support optique, le dispositif d'affichage étant prévu sur un côté du support optique qui est opposé à une position souhaitée du chirurgien, dans lequel
la forme de la deuxième section comprend une première sous-section (142) qui s'étend latéralement vers l'extérieur depuis le point de suspension, une deuxième sous-section (144) qui s'étend vers le bas depuis la première sous-section, et une troisième sous-section (146) qui revient vers l'intérieur depuis la deuxième sous-section vers le support optique et **caractérisé en ce que** la deuxième sous-section (144) s'étend vers le bas sur au moins 20 cm.
